Europäisches Patentamt

European Patent Office

Office européen des brevets

(11) Publication number: **0 363 041**

**A1**

(12) **EUROPEAN PATENT APPLICATION**

(21) Application number: 89309527.3

(22) Date of filing: 19.09.89

(51) Int. Cl.5: **G01N 33/94** , **G01N 33/577** , **C12P 21/00**

(30) Priority: 21.09.88 JP 234826/88
25.10.88 JP 267207/88

(43) Date of publication of application:
**11.04.90 Bulletin 90/15**

(84) Designated Contracting States:
**DE FR GB**

(71) Applicant: UBE INDUSTRIES, LTD.
12-32, Nishihonmachi 1-chome
Ube-shi, Yamaguchi-ken(JP)

(72) Inventor: Uda, Taizo c/o Ube Research
Laboratory
UBE INDUSTRIES, LTD. 1978-5 Oaza Kogushi
Ube-shi Yamaguchi-ken(JP)
Inventor: Itoh, Yukikatsu c/o Ube Research
Laboratory
UBE INDUSTRIES, LTD. 1978-5 Oaza Kogushi
Ube-shi Yamaguchi-ken(JP)
Inventor: Usagawa, Takashi c/o Ube Research
Laboratory
UBE INDUSTRIES, LTD. 1978-5 Oaza Kogushi
Ube-shi Yamaguchi-ken(JP)
Inventor: Hongyo, Kenichi c/o Ube Research
Laboratory
UBE INDUSTRIES, LTD. 1978-5 Oaza Kogushi
Ube-shi Yamaguchi-ken(JP)

(74) Representative: Brock, Peter William et al
URQUHART-DYKES & LORD 91 Wimpole
Street
London W1M 8AH(GB)

(54) Monoclonal antibody to morphine, preparation of monoclonal antibody, assaying kit and assaying method of morphine.

(57) There are disclosed a monoclonal antibody to morphine produced by the hybridoma strain obtained by cell fusion of mouse lymphocytes and mouse myeloma cells, characterized in that it does not substantially react with codeine but has a very high specific reactivity with morphine, a method for preparing a monoclonal antibody to morphine, which comprises culturing the hybridoma strain as mentioned above, an assaying kit of morphine which comprises essentially:

(a) a carrier having an antibody having a very high specific immunoreactivities with morphine and normorphine carried thereon; and

(b) a carrier having normorphine carried thereon, and an assaying method of normorphine, which comprises reacting morphine in a sample to be assayed and normorphine carried on a carrier competitively with the antibody carried on the carrier by use of the assaying kit of morphine as mentioned above.

Xerox Copy Centre

## Monoclonal antibody to morphine, preparation of monoclonal antibody, assaying kit and assaying method of morphine

### BACKGROUND OF THE INVENTION

This invention relates to a monoclonal antibody having a very high reactivity with morphine which is an analgesic, a method for preparing the monoclonal antibody and a hybridoma strain producing the monoclonal antibody.

The present invention further relates to an assaying kit and an assaying method of morphine.

In recent years, as one of the therapeutical method of terminal cancer patients, there has been employed the method to release pain by administration of morphine. According to such a method, if a dose of morphine administered is too small, there is no analgesic effect, while if the dose is too much, the side effect will become too great. Accordingly, for determining the administration dose for maintaining it at an adequate concentration, it is necessary to use the method which enables monitoring its plasma concentration conveniently and rapidly.

As such a simple method, there have been known in the art an immunological assaying method such as radioimmunoassay employing of enzyme immunoassay by use of a polyclonal antibody [Spector and Parker, Science, 168, 1347 - 1348, 1970; Catlin et al., Clinical Chemistry, 19, 2, 216 -220, 1973; Gintzler et al., European Journal of Pharmacology, 38, 149 - 156, 1976; and Findlay et al., Research Communications in Chemical Pathology and Pharmacology, 17, 4, 595 - 603, 1977] or a monoclonal antibody [Glasel et al., Molecular Immunology, 20, 12, 1419 - 1422, 1983; and Sawada et al., Molecular Immunology, 25, 9, 937 -943, 1988].

However, since most of the polyclonal antibodies and both of monoclonal antibodies used in these assays have cross-reactivities with codeine (antitussive), there has been known no monoclonal antibody which exhibits a very high specific reactivity with morphine so as to enable accurate assay of the morphine level in blood even when codeine may exist in blood but exhibits no reactivity with codeine.

Also, when assaying a large number of test samples, these assaying methods require a considerable time and the assaying procedures are also complicated.

Accordingly, in monitoring of plasma concentration, it is required to improve the assaying method so that assay can be done further conveniently and rapidly.

### SUMMARY OF THE INVENTION

An object of the present invention is to provide a monoclonal antibody having a very high specific reactivity with morphine which is an analgesic, a method for preparing the monoclonal antibody and a hybridoma strain producing the monoclonal antibody.

Another object of the present invention is to provide an assaying kit comprising essentially a carrier having an antibody having a very specific immunoreactivity with morphine and normorphine carried thereon and a carrier having normorphine carried thereon, and an assaying method of morphine by use of the assaying kit.

The present inventors have studied intensively in order to solve the above problems, and consequently found that a monoclonal antibody exhibiting a very high specific reactivity with morphine which is an analgesic can be obtained by culturing a hybridoma strain obtained by fusion of mouse lymphocytes with mouse myeloma cells, to accomplish the present invention.

The present inventors have further found that morphine can be assayed more conveniently and rapidly than the assaying method of the prior art by assaying morphine by use of an assaying kit of morphine to accomplish the present invention.

More specifically, the present invention concerns a monoclonal antibody to morphine, characterized by having a very high specific reactivity with morphine, although not substantially reactive with codeine, produced by a hybridoma strain obtained by cell fusion of mouse lymphocytes and mouse myeloma cells.

Further, the present invention concerns a method for preparing the monoclonal antibody to morphine, which comprises culturing the above hybridoma strain.

Further, the present invention concerns the above hybridoma strain.

A fourth invention is an assaying kit to be used in assay of morphine comprising essentially:

2

(a) a carrier having an antibody having a very specific immunoreactivity with morphine and normorphine carried thereon; and

(b) a carrier having normorphine carried thereon.

A fifth invention concerns an assaying method of morphine, which comprises reacting the morphine in the assay sample and the normorphine carried on the carrier competitively with the antibody carried on the carrier.

A sixth invention concerns an assaying method of morphine, which comprises reacting a normorphine-bound macromolecular protein immobilized on a solid phase support and morphine in a sample to be assayed competitively with a reagent comprising a monoclonal antibody having very high specific immunoactivity with morphine labelled with an enzyme.

A seventh invention is an assaying kit to be used in assay of morphine comprising essentially:

(a) a normorphine-bound macromolecular protein; and

(b) a reagent comprising a monoclonal antibody having a very high specific immunoreactivity with morphine labeled with an enzyme.

## BRIEF DESCRIPTION OF THE DRAWING

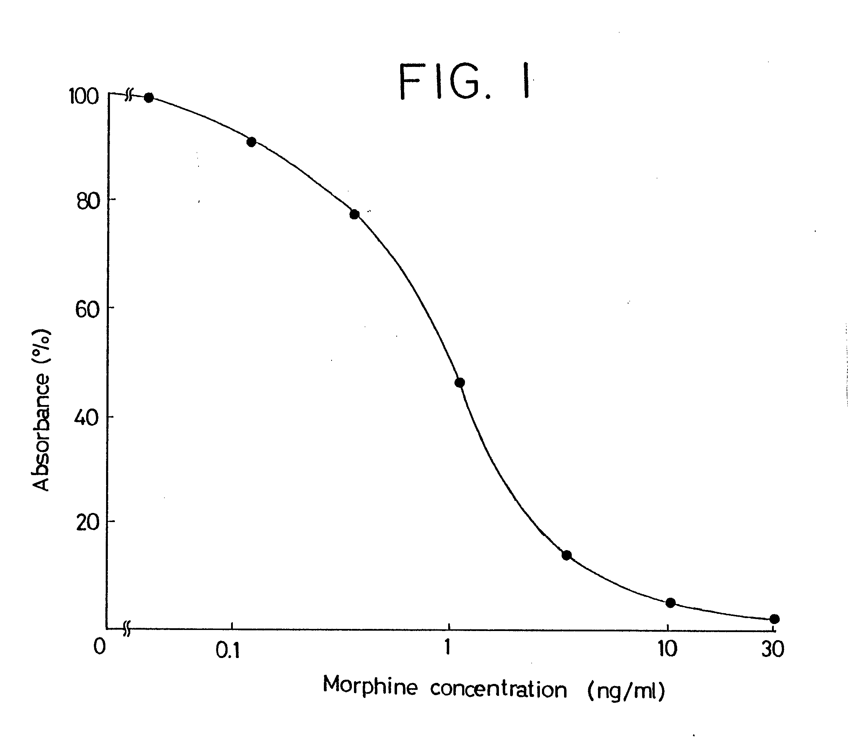

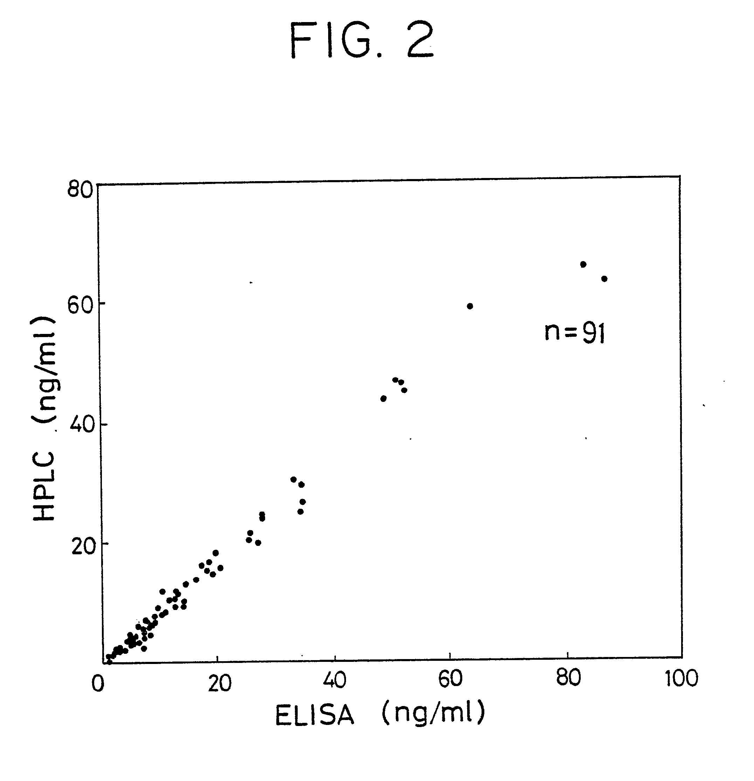

Fig. 1 shows the standard curve of morphine prepared by use of "enzyme-labeled antibody", and Fig. 2 shows the relationship of the test results measured by ELISA method and HPLC method.

## DESCRIPTION OF THE PREFERRED EMBODIMENTS

In the following, the present invention will be described in detail.

The monoclonal antibody of the present invention is produced by the hybridoma strain obtained by cell fusion of mouse lymphocytes and mouse myeloma cells, and has a very high specific reactivity with morphine.

The mouse lymphocytes to be used in the present invention are not particularly limited, provided that a hybridoma strain capable of producing a monoclonal antibody having a very high specific reactivity with morphine by cell fusion with mouse myeloma cells can be obtained, but preferably lymphocytes obtained by immunization with an appropriate immunogen may be used.

As the appropriate immunogen to be used for immunization of mouse in the present invention, any immunogen capable of giving a monoclonal antibody having a very high specific reactivity with morphine can be used without particular limitation, including, for example, morphine, salts of morphine, morphine bound to a macromolecular carrier having a molecular weight of 10,000 or more, normorphine, normorphine bound to a macromolecular carrier having a molecular weight of 10,000 or more, preferably normorphine bound to a macromolecular carrier having a molecular weight of 10,000 or more. As the macromolecular carrier having a molecular weight of 10,000 or more, there may be employed biological macromolecular compounds such as bovine serum albumin (BSA), ovalbumin (OVA), key-hole limpet hemocyanine (KLH), immunoglobulins, etc., and polyamino acids such as poly-L-lysine (PLL), etc.

The monoclonal antibody having a very high specific reactivity with morphine of the present invention has a high specific reactivity with morphine, but has substantially no reactivity recognized with cocaine, codeine, dihydrocodeine, ethylmorphine, fentanyl, methadone, M-3-G (morphine-3-glucronide), etc. The monoclonal antibody having such a specificity can be obtained by, for example, stationary cultivation in a flask by use of lymphocytes cultivation medium in general of MO-2 strain (FERM-P No. 1910), MO-3 strain (FERM-P No. 1911), MO-4 strain, MO-5 strain (FERM-P No. 1912), MO-6 strain, etc. which are hybridoma strains obtained by fusion of lymphocytes obtained from the immunized mouse in which normorphine bound to an immunoglobulin which is a biological macromolecule is used as the immunogen and myeloma cells of mouse. Further, when a monoclonal antibody having substantially no reactivity recognized with M-6-G (morphine-6-glucronide) is required, a monoclonal antibody having further higher specificity can be obtained by culturing MO-3 strain, MO-4 strain, MO-5 strain or MO-6 strain.

Preparation of such hybridoma strain can be performed according to the method known in the art, for example, the method of Milstein and Köhler [Nature, 256, 495 (1976)].

The outline of the preferred method for preparing such a hybridoma strain is to be described successively below.

3

Preparation of monoclonal antibody-producing hybridoma strain

(i) Preparation of immunogen and antigen for assay:

The immunogen to be used for mouse can be prepared by, for example, converting a compound such as normorphine or a salt of normorphine into a N-(4-bromobutyl) derivative by use of N-(4-bromobutyl)-phthalimide and binding it to a macromolecular carrier with a molecular weight of 10,000 or more (including biological macromolecules such as BSA, OVA, KLH, immunoglobulin, etc., and polyamino acid such as PLL as preferable examples) with the use of a carbo diimide such as 1-cyclohexyl-3-(2-morpholinoethyl)-carbodiimide metho-p-toluenesulfonate (hereinafter abbreviated as "CMEC"), 1-ethyl-3-(dimethylaminopropyl)carbodiimide hydrochloride (hereinafter abbreviated as "EDPC"), etc.

On the other hand, as the antigen for assay, one prepared in the same manner as in the above preparation method of the immunogen by use of a macromolecular carrier and a carbodiimide which are different from the macromolecular carrier and the carbodiimide used in preparation of the immunogen are used.

(ii) Preparation of immunized mouse lymphocytes:

The immunization method of mouse can be performed by administering the immunogen of the above (i) (10 - 400 μg) dissolved in PBS (Dulbecco's phosphate buffered saline) to mouse once or for several times at intervals of several weeks.

The first immunization can be also performed without administration of an adjuvant (immunization promoting substance containing alum, dead tuberclosis bacterial cells, nucleic acid, etc.), but it is preferred to administer an emulsion prepared by use of an adjuvant.

Lymphooytes can be obtained from blood, lymph node, spleen, etc. several days after the final immunization after confirmation of sufficient antibody value of the immunized mouse, but preferably from spleen.

(iii) Preparation of myeloma cells:

For cell fusion, myeloma cells such as MPC-11, P3-X63-Ag8·653 (653), P3-X63-Ag8-U1 (P3U1), P3-NS-1 (NS-1), SP2/0-Ag14(SP2/0), etc. derived from mouse, and 210.RC Y3.Ag1.2.3 (Y3), etc. derived from rat can be used, but it is preferred to use myeloma cells which will not produce by secretion the antibodies out of the cells such as 653, P3U1, NS-1, SP2/0, etc.

(iv) Cell fusion:

Cell fusion can be performed by mixing well the mouse lymphocytes immunized as described above and the above myeloma cells at a ratio of cell number of (5 to 20): 1 with the use of a cell suspension which gives no trouble in cell fusion, for example, a solution of medium components for culturing lymphocyte generally used (medium components such as MEM, DMEM, McCoy, RPMI1640, etc.), isotonic buffer, etc., and adding to the pellet after centrifugation (cell mass), HVJ (Sendai virus) or PEG (polyethylene glycol) solution, but it is preferred to use a PEG solution, more preferably a 30 to 60 % by weight of PEG solution with an average molecular weight of 1,000 to 8,000. At this time, for promoting cell fusion, corhitine, dimethyl sulfoxide, poly-L-arginine, etc. can be also added in suitable amounts.

As the myeloma cells to be used for cell fusion, those derived from an animal different in species from the immunized mouse can be also used, but in view of the aspects of the production amount of the antibody of the monoclonal antibody producing hybridoma strain obtained and its stability, it is preferred to use a myeloma cell of the same species as the immunized mouse, more preferably of the same strain.

(v) Selection of hybridoma:

Selection of hybridoma can be performed by culturing the cells after manipulation of the cell fusion in a HAT medium (medium containing hypoxanthine, aminopterin, thymidine, fetal calf serum (FCS); as the medium components, the medium components for culturing lymphocyte generally employed can be used).

The hybridoma is cultured by placing cells in a number suitable for screening of antibody producing wells in each well (culturing well) of the culturing plate, and at this time, it is possible to use a growth promoting substance of the hybridoma or cells producing it (e.g. lymphocytes derived from thymus, spleen, lymph node, etc.) as the feeder cells, if necessary.

The hybridoma selected by growth in a HAT medium is cultured in a HT medium (medium containing hypoxanthine, thymidine, FCS; as the medium components, medium components for culturing lymphocytes generally employed can be used) for several days, and further cultured in a medium for lymphocyte cultivation containing FCS generally employed.

(vi) Selection of antibody-producing hybridoma:

Assay whether the hybridoma obtained in the above (v) has produced the desired antibody or not can be performed by, for example, the ELISA method (enzyme-linked immunosorbent assay), the plaque formation method, the agglutination reaction method, RIA (the method by use of radioisotope), the indirect fluorescent antibody assay (IFA), etc., but when the number of inspections is very many, it should be preferably performed by the ELISA method.

The ELISA method is conducted as described below.

Into each well (assay well) of the ELISA plate having the antigen for assay prepared in (i) fixed thereon was added the hybridoma culture supernatant, followed by standing stationarily for a predetermined time. Then, to these assay wells is added an enzyme-labelled antibody capable of reacting with and being bound through the antibody derived from an animal bound to each assay well washed (as the enzyme to be used for labelling, for example, peroxidase, alkaline phosphatase, $\beta$-galactosidase, etc. may be included; the antibody to be labelled is not particularly limited, provided that it can be bound through the reaction only with the antibody derived from the animal bound to the assay well, including antisera obtained form mouse, rat, rabbit, goat, etc., or monoclonal antibodies produced by the hybridoma strains prepared by use of mouse cells, etc.). Next, these assay wells are washed, and the enzyme activity is assayed by addition of a substrate solution corresponding to the enzyme employed. If an enzyme activity is recognized, it can be understood that the hybridoma capable of producing the desired antibody has existed in the culture well from which the culture supernatant is taken.

Thus, cell growth is recognized, and also the hybridoma producing the antibody can be obtained.

(vii) Cloning of hybridoma:

The hybridoma in the culture well recognized of antibody production can be cloned according to such method as the limiting dilution method, the single cell manipulation method (the method in which one hybridoma is placed in one well under an inverted microscope), the method in which colonies are picked up by use of a soft agar, the method by use of FACS (Fluorescent Activated Cell Sorter), etc. At this time, the antibody producing hybridoma found in (vi) is cultured according to any of the above cloning methods, and by use of the supernatant of the culture well in which its growth is recognized, the antibody producing well is screened according to the same ELISA method as performed in selection of the antibody producing hybridoma of (vi).

Thus, a hybridoma which produces a monoclonal antibody having high specificity for morphine as well as high antibody value can be obtained by selection.

Preparation method of monoclonal antibody

The monoclonal antibody having high specificity for morphine as well as high antibody titer can be produced by culturing the hybridoma strain obtained in the above (vii) in a flask or culturing it intraperitoneally in an animal.

Production of said monoclonal antibody by the hybridoma strain obtained in the above (vii) in a flask can be performed by culturing in, for example, a medium for culturing lymphocyte generally employed containing 0 to 20 % fetal calf serum (e.g. medium containing medium components such as MEM, DMEM, McCoy, RPMI1640, etc.) until the cell concentration reaches the upper limit. At this time, said monoclonal antibody is contained in the culture supernatant obtained by the centrifugal operation.

On the other hand, production of said monoclonal antibody by intraperitoneal cultivation in an animal of the hybridoma strain obtained in the above (vii) in an animal can be also performed by use of an animal heterologous from the animal from which the cells used for cell fusion are derived, but it is preferable to use an animal of the same species, more preferably of the same strain.

Production of said monoclonal antibody having high specificity for morphine as well as high antibody titer according to such method can be performed by administering a substance which lowers such immunization ability of an appropriate animal such as mouse, rat, hamster, etc., for example, a mineral oil such as pristane, etc., intraperitoneally into the animal, administering several weeks later $10^6$ to $10^7$ cells of the hybridoma strain obtained in the above (vi), and permitting the strain cell to be grown intraperitoneally at high density within several weeks. At this time, said monoclonal antibody is contained in the ascites supernatant obtained by the centrifugal operation. Its antibody concentration is about 10 to 1000-fold of the antibody concentration of the culture supernatant obtained by culture within a flask.

Said monoclonal antibody obtained by cultivation of the hybridoma strain in a flask or intraperitoneally in an animal can be purified by performing salting out, dialysis, ion exchange chromatography, affinity chromatography, etc. applied to purification methods of proteins in general to give a monoclonal antibody of high purity.

Said monoclonal antibody obtained as described above has a very high specific reactivity with morphine.

The morphine assaying kit to be used in the assaying method of morphine of the present invention comprises essentially a carrier having an antibody having a very high specific immunoreactivity with morphine and normorphine carried thereon (hereinafter abbreviated as "antibody carrier") and a carrier having normorphine (analogous compound of morphine) carried thereon (hereinafter abbreviated as "normorphine carrier"). For assay of morphine, in addition to these reagents, the reaction plate which becomes the place for these reactions, known amounts of morphine solutions (hereinafter abbreviated as "standard morphine solutions"), a stirring rod, etc. are also required, and these may be either assembled previously in the assaying kit of morphine, or prepared before assay of morphine.

The antibody to morphine and normorphine which can be used in preparation of the "antibody carrier" in the assaying kit of morphine in the present invention is not particularly limited, provided that it has a very high specific immunoreactivity with morphine and normorphine, and an antiserum or a monoclonal antibody, etc. can be used, preferably a monoclonal antibody. Examples of such monoclonal antibody having a very high specificity for morphine and normorphine may include one comprising at least one of monoclonal antibodies produced by hybridoma strain produced from mouse obtained by immunization of normorphine as disclosed in Japanese Patent Application No. 234826/1988 [e.g. MO-2 strain (FERM-P No. 1910), MO-3 strain (FERM-P No. 1911), MO-4 strain, MO-5 strain (FERM-P No. 1912), MO-6 strain, etc.].

When the "antibody carrier" to be used during assay of morphine in the present invention is powder, it is dispersed in an appropriate solvent such as water, a buffer (e.g. phosphate buffer, Tris-HCl buffer, etc.) at an appropriate concentration before. use.

In preparation of the "antibody carrier" or the "normorphine carrier" in the assaying kit of the present invention, the material of the carrier for carrying the antibody or normorphine thereon may include polyethylene, polystyrene, polypropylene, nitrocellulose, glass, nylon, PMMA (polymethyl methacrylate), styrene-divinyl benzene copolymer, styrene-butadiene copolymer, etc., preferably polystyrene.

A shape of these carriers used may be sphere, rod, needle, cube, etc., preferably sphere.

A size of these carriers used may be 0.01 to 5 $\mu$m, particularly 0.1 to 3 $\mu$m, further preferably 0.2 to 2 $\mu$m. Also, the size and the shape of the carrier employed may be preferably uniform. These carriers can be also suitably colored in view of the color of the reaction plate used during assay.

The "antibody carrier" in the present invention can be prepared as described below.

An antibody having a very high specificity with morphine is dissolved in a buffer controlled to around neutral (e.g. phosphate buffer, Tris-HCl buffer, etc.) to prepare a solution of an appropriate antibody concentration, and the solution can be subjected to the contact reaction according to the method of stationary standing or shaking for a certain period of time to have the antibody carried on the carrier. The heating temperature during the contact may be 10 to 50 °C, preferably 20 to 40 °C. Also, the heating temperature at this time may be preferably longer as the temperature in the above heating temperature range is lower (for example, sufficiently 2 to 4 hours at 30 to 50 °C, and requires 4 to 10 days at 10 to 29 °C; when heated for a long time; it is preferred to add a preservative for protein such as sodium azide, sodium ethylmercurythiosulfate, etc.).

The "antibody carrier" thus obtained can be also stored under the suspension state as such at a low temperature (e.g. 2 to 10 °C), but preferably a preservative for protein such as sodium azide, sodium ethylmercurythiosulfate, etc., or a stabilizing substance for protein such as bovine serum albumin (BSA) can be also added in necessary amount.

The "normorphine carrier" in the present invention can be prepared by carrying a bound product obtained by binding normorphine chemically with a macromolecular protein having a molecular weight of 10,000 or more on a carrier.

As the macromolecular protein having a molecular weight of 10,000 or more to be used in preparation of the "normorphine carrier" in the present invention, there may be included biological macromolecules such as BSA, OVA, KLH, γ-globulin, etc., and polyamino acids such as PLL, etc.

Binding between normorphine and the macromolecular protein in preparation of the "normorphine carrier" can be effected by binding the product obtained by converting normorphine into N-(4-bromobutyl) derivative by use of N-(4-bromobutyl)phthalimide to the above macromolecular protein with the use of a carbodiimide such as EDPC, CMEC, etc. as mentioned above.

The "normorphine carrier" can be prepared by subjecting the bound product of normorphine and the macromolecular protein thus obtained as such or after purification by gel filtration by use of Sephadex, Sephacryl (both trade names), etc. to the contact reaction with a carrier according to the method of stationary standing or shaking for a certain period of time to have the antibody carried on the carrier. The heating temperature during the contact may be 10 to 50 °C, preferably 20 to 40 °C. Also, the heating time at this time may be preferably longer as the temperature during the above temperature heating range is lower (for example, sufficiently 2 to 4 hours at 30 to 50 °C, requiring 4 to 10 days at 10 to 29 °C; when heating is carried out over prolonged time, a preservative for protein such as sodium azide, sodium ethylmercury-thiosulfate may be preferably added).

The "normorphine carrier" thus obtained can be also stored at a low temperature (e.g. 2 to 10 °C) under the suspension state as such, but preferably a preservative for protein such as sodium azide, sodium ethylmercurythiosulfate, etc., or a stabilizing substance for protein such as BSA, etc. can be also added in necessary amount.

As the assay sample to be used in assay of morphine of the present invention, there can be used human body fluids such as human urine, blood, serum, etc. These assay samples can be used either with or without dilution for assay.

The reaction plate which becomes the place for the reaction to be used in assay of morphine of the present invention may include glass plate, plastic plate, plate (made of wood, paper, etc.) coated with a water-proof substance, but it is preferred to use a plate made of a paper coated with a plastic. The color of the reaction plate is not particularly limited, provided that the agglutinated substance formed as the result of the reaction between the "normorphine carrier" and the "antibody carrier" can be clearly confirmed, but, for example, it should be preferably be made black, when the agglutinated substance is white.

As the rod for stirring the reaction mixture to be used in assay of morphine of the present invention, any waterproof rod may be available without particular limitation, but preferably a rod made of a plastic or coated with a plastic is to be used. A size of the rod is not particularly limited, so long as the object of the present invention can be accomplished, but, for example, a rod with a length of 3 to 10 cm and a diameter of 0.1 to 5 mm can be used.

Having the reaction plate, the stirring rod for the reaction mixture, the assay sample and "standard morphine solutions" prepared as described above, by use of the "antibody carrier", the "normorphine carrier" which are reagents of the assaying kit of morphine, morphine in the assay sample can be assayed via the respective steps of (i) to (ii) as described below (assay of normorphine can be conducted according to the same assaying operations in the case of morphine except for using a normorphine assaying sample and known amounts of normorphine solutions).

(i) The step of placing certain amounts of "antibody carrier", "normorphine carrier" and sample to be assayed or "standard morphine solution":

Each of "antibody carrier", "normorphine carrier", a sample to be assayed or "standard morphine solution" is placed on the reaction plate in a certain amount (for example, 5 to 50 μl) as one set with positional intervals apart from each other so that they can be readily stirred. At this time, it is preferred to place a set comprising ["antibody carrier", "normorphine carrier" and a sample to be assayed] and a set comprising ["antibody carrier", "normorphine carrier" and "standard morphine solution"] at appropriate positional intervals apart from each other on the same reaction plate.

(ii) The step of reacting the respective solutions on the above reaction plate of (i) with each other by stirring each set:

Each solution was quickly stirred with a stirring rod, and the reaction is carried out while moving back and forth, left and right at a certain temperature (e.g. room temperature, etc.), for a certain period of time (e.g. 1 to 2 minutes), and the agglutination of each set formed by the reaction between the "antibody carrier" and the "normorphine carrier" is observed. At this time, by observing in comparison between the agglutination in the case of employing the set by use of the "standard mor phine solution" and the agglutination in the case of employing the set by use of the sample to be assayed, the morphine content in the sample to be assayed can be known.

Of the procedures in the morphine assaying operation, in the step of carrying out the reaction by stirring the respective reagents on the reaction plate for each set in (ii), competitive reactions occur between the normorphine in the "normorphine carrier" or the morphine in the "standard morphine solution" and the antibody to morphine carried on the "antibody carrier", and the agglutination reaction occurs between the "antibody carrier" and the "normorphine carrier", whereby the morphine content in the sample can be assayed rapidly and at high sensitivity by comparative observation of the agglutination in the case of the set by use of the "standard morphine solution" and the the agglutination in the case of the set by use of the assay sample.

The morphine (hereinafter abbreviated to as "MO") assay kit to be used in the method for assay MO of the present invention comprises essentially a normorphine bound macromolecular protein (hereinafter abbreviated to as "NM bound protein" and a reagent having the monoclonal antibody having very high specific immunoreactivity with NM labelled with an enzyme (hereinafter abbreviated to as "enzyme-labeled antibody"). For assay of MO, in addition to these reagents, solid phase support, washing solution, blocking solution, MO or a substitute for MO having the substantially the same inhibition activity to MO antibody for preparation of calibration curve of MO, substrate solution of enzyme, etc. are also required, and these may be previously assembled in the assay kit of MO, or they may be prepared before assay.

Preparation of the "NM bound protein" in the MO assay kit of the present invention is required for permitting the NM immobilized through a macromolecular protein onto a solid phase support to react with the reagent having the monoclonal antibody having very high specific immunoreactivity with MO labeled with an enzyme. As the macromolecular protein to be used for its preparation, there can be included bovine serum albumin (BSA), ovalbumin (OVA), key-hole limpet hemocyanine (KLH), γ-globulin, etc.

In preparation of the "support of morphine" of the present invention, firstly normorphine is converted into a N-(4-bromobutyl) derivative by use of N-(4-bromobutyl)phthalimide and then to the resulting product is added hydrated chloral to effect dephthalamine reaction to give the aimed N-(4-aminobutyl)normorphine (hereinafter abbreviated to as "ABNM"). Subsequently, this ABNM is reacted with a carbodiimide such as EDPC, CMEC, etc. to bind with the above macromolecular protein.

Although the thus obtained binder of normorphine and macromolecular protein can be used as such, but it is preferred to purify this according to gel filtration by use of Sephadex, Sephacryl (both trade names), etc. before use.

The "NM bound protein" fraction obtained by such gel filtration can be also used as such (when the protein concentration is low, it is concentrated through an ultrafiltration membrane, etc. to a desired concentration, while when the protein concentration is high, it is diluted to a desired concentration), but it is preferably dialyzed with a buffer adjusted to pH around neutral (e.g. phosphate buffer or Tris-hydrochloride buffer), stored after lyophilization or filtration through sterilizing filter, and then used as a "NM bound protein" solution, if desired, at a protein concentration of 0.1 to 100 μg/ml, preferably 1 to 20 μg/ml.

Also, if necessary, a preservative for protein such as sodium azide can be added in a small amount to the "NM bound protein".

In the preparation of the "enzyme-labeled antibody" of the MO assay kit of the present invention, the enzyme for labeling the antibody may include at least one selected from oxidoreductase such as peroxidase, catalase, glucose oxidase, lactate oxidase, alcohol oxidase, monoamine oxidase, β-galactosidase, etc., and phosphate hydrolases such as alkaline phosphatase, etc.

As the antibody labeled with the above enzyme, there may be included the monoclonal antibodies produced by the strains MO-2 strain (FERM-P No. 1910), MO-3 strain (FERM-P No. 1911), MO-4 strain, MO-5 strain (FERM-P No. 1912), MO-6 strain, etc. which are hybridoma as described above.

The "enzyme-labeled antibody" of the present invention can be prepared by binding at least one monoclonal antibody as mentioned above with at least one enzyme as mentioned above according to the one step method by use of glutaraldehyde [Immunochemistry, 6, 43, (1969)] or the two step method [Immunochemistry, 8, 1175 (1971)], the periodic acid oxidation method [Method in Enzymology, 37, 133 (1975], the maleimide method [Journal of the Biochemistry, 78, 235 (1975)], etc., of which the latter two methods are preferred.

This can be used as such for assay of MO, but for the purpose of further enhancing assay sensitivity of MO, it is preferred to use the "enzyme-labeled antibody" obtained by purifying this by gel filtration with the use of Sephadex, Sephacryl (both trade names), etc. as the assay reagent for MO.

Although the "enzyme-labeled antibody" obtained by such gel filtration can be also used as such, it is preferably dialyzed with a buffer adjusted to pH at around neutral (e.g. phosphate buffer of Tris-hydrochloride buffer), stored after lyophilization or filtration through sterilizing filter, and then used as an "enzyme-labeled antibody" solution, if desired, at a protein concentration of 0.01 to 100 μg/ml, preferably 0.1 to 10 μg/ml.

The solid phase support to be used in the MO assay method of the present invention is required as the support for immobilizing the "NM bound protein" of the MO assay kit.

The shape of the solid phase support which can be used for immobilizing the NM bound macromolecular protein may include, for example, plate, tube, beads, membrane, etc. for immunoassay, and its material may include, for example, polyethylene, polystyrene, polypropylene, nitrocellulose, glass, etc.

The washing solution to be used in the MO assay of the present invention is required for washing away the "NM bound protein" not immobilized on the solid phase support after "immobilization of a certain amount of the "NM bound protein" thereon, or washing away the unreacted substance after the competitive reaction between the MO in the sample to be assayed and the "NM bound protein" immobilized on the solid phase support with the "enzyme-labeled antibody". Also, the washing solution may be used as a diluent for a sample to be assayed or a solvent for preparing the blocking solution.

As the washing solution to be used for such purposes, there can be included, for example, water, a buffer adjusted to the pH during the reaction (buffer such as phosphate buffer, Tris-hydrochloride buffer, etc.), the above buffer containing 0 to 3 % by volume of a surfactant such as Tween 20 and Tween 60 (both trade names), but it is preferred to use a buffer containing 0.005 to 0.8 % by volume of the above surfactant adjusted to the pH during the reaction.

The blocking solution to be used in the MO assay method of the present invention for preventing non-specific binding of the "enzyme-labeled antibody" to the solid phase support having no "NM bound protein" immobilized thereon.

The blocking solution to be used for such purposes can be prepared by dissolving a macromolecular protein such as BSA, OVA, KLH, γ-globulin, etc. in a washing solution adjusted to the pH during the reaction, and the concentration of these macromolecular proteins may be 0.1 to 10 weight/volume %, preferably 0.1 to 2 weight/volume %, and when it is preferred by use of sera of various animals, the concentration is made 1 to 50 volume/volume %, preferably 10 to 20 volume/volume %, with the use of the above washing solution.

Also, if necessary, the blocking solution can also be contain a necessary amount of a preservative for protein such as sodium azide, sodium ethylmercurithiosalicylate, etc. added therein.

As the sample to be used in the MO assay method of the present invention, human body fluid such as human urine, blood, serum, etc. can be used.

In the assay of MO, MO is assayed by use of these samples to be assayed diluted with the above washing solution within the range at which MO can be assayed.

As the substrate solution for enzyme to be used in the MO assay method of the present invention (hereinafter abbreviated to as "substrate solution"), a buffer containing the substrate of $H_2O_2$ with which the enzyme used for the label of the antibody in the "enzyme-labeled antibody" reacts, and a substance which is colored by the product formed by the enzymatic reaction such as O-phenylenediamine, 2,2'-amino-bis(3-ethylbenzothiazoline-6-sulfonic acid) (ABTS), etc.

As described above, the solid phase support, the washing solution, the blocking solution, the MO solution for preparation o the calibration curve of MO and the "substrate solution" are prepared, and by use of the "NM bound protein", the "enzyme-labeled antibody", which is the assay kit of MO, the MO in a sample to be assayed can be assayed via the respective steps as shown below.

(1) The steps of immobilizing a certain amount of the "NM bound protein" on the solid phase support:

A certain volume of a solution of the "NM bound protein" is brought into contact with the solid phase support for a certain period of time. The temperature during contact is not particularly limited, so long as the "NM bound protein" is not frozen or boiled, but may be preferably 2 to 40 °C.

(2) The step of removing the "NM bound protein" not immobilized on the solid phase support:

After the "NM bound protein" is brought into contact with the solid phase support for a certain period of time, the "NM bound protein" solution is removed, and further the remaining "NM bound protein" not immobilized on the solid phase support is removed by washing with the washing solution for several times.

(3) The steps of preventing non-specific binding of the "enzyme-labeled antibody" to the solid phase support surface having no "NM bound protein" immobilized thereon:

A certain volume of the blocking solution is brought into contact with the above solid phase support having the "NM bound protein" immobilized thereon for a certain period of time. The temperature during contact is not particularly limited, so long as the blocking solution is not frozen or boiled, but may be preferably 2 to 40 °C.

(4) The step of allowing the MO in a sample to be assayed and the "NM bound protein" immobilized on the solid phase support to react competitively with the "enzyme-labeled antibody":

Equal volume of the sample to be assayed and the "enzyme-labeled antibody" solution are allowed to contact and react with the "NM bound protein" immobilized on the solid phase support as described above. The sample to be assayed and the "enzyme-labeled antibody" solution may be either subjected to the contact reaction with the "NM bound protein" immediately after mixing, or alternatively each successively contacted with the "NM bound protein", followed immediately by stirring to effect the reaction.

(5) The steps of removing the "enzyme-labeled antibody" not immobilized on the solid phase support through the "NM bound protein":

The above mixture of the sample to be assayed and the "enzyme-labeled antibody" solution is removed, and the mixture of the sample to be assayed and the "enzyme-labeled antibody" solution remaining without binding to the solid phase support through the "NM bound protein" is removed by washing with the washing solution for several times.

(6) The step of allowing the "enzyme-labeled antibody" immobilized on the solid phase support through the "NM bound protein" with the "substrate solution":

As the "substrate solution" to be used here, one corresponding to the enzyme used for labeling of the "enzyme-labeled antibody" (including the substance which is colored when the enzyme reaction occurs) is added.

Its certain volume is allowed to react with the "enzyme-labeled antibody" immobilized on the solid phase support through the "NM bound protein" as described above for a certain period of time, and the enzyme reaction should be preferably stopped with the use of an acid such as $H_2SO_4$, a base such as NaOH or an enzyme inhibitor. The reaction temperature will pose no problem within the optimum temperature range for the enzyme employed, but may be preferably 20 to 35 °C.

(7) The step of assay the absorbance of the reaction mixture after the above enzyme reaction:

The absorbance of the reaction is assayed at the wavelength when coloration of the reaction mixture after the above enzyme reaction exhibits the maximum absorbance.

A calibration curve can be prepared from the results obtained by use of the known amounts of MO in place of the sample to be assayed as described above, from which the MO content in the sample to be assayed can be known.

In the step (4) of adding the "enzyme-labeled antibody" solution and the sample to be assayed of the procedure of the MO assay operation, the competitive reactions of the MO of the "NM bound protein" immobilized on the solid phase support and the MO in the sample to be measured with the "enzyme-labeled antibody" occur, whereby the MO content in the sample can be assayed rapidly and at high sensitivity.

## EXAMPLES

In the following, Examples of the present invention are described in detail. These Examples are not intended to limit the scope of the present invention at all.

### Example 1

[Preparation of immunogen and antigen for assay]

In 20 ml of dioxane was dissolved 271 mg of normorphine (hereinafter abbreviated as NM), and to the solution were added a solution of 340 mg of N-(4-bromobutyl)phthalimide dissolved in 5 ml of ethanol and 600 mg of sodium carbonate, followed subsequently by heating under reflux for 20 hours. After the inorganic precipitates were removed from the reaction mixture by filtration, the filtrate was evaporated under reduced pressure. The residue obtained was dissolved in an extremely small amount of ethyl acetate and the solution was applied onto a silica gel column [20 mm$\phi$ x 300 mmL; Kieselgel 60 (trade name) (70 to 200 mesh ASTM)]. The column was eluted with 60 ml of ethyl acetate/methanol (9 : 1) and subsequently with ethyl acetate/methanol (5 : 1) and fractionated into each 20 ml of fractions. The fractions were analyzed by TLC (Kieselgel 60 (trade name); developing solvent was ethyl acetate/methanol/conc. ammonia water = 85 : 10 : 5) and the fractions containing only the desired product were collected, and the solvent was removed under reduced pressure. The residue obtained was dissolved in 20 ml of ethanol and then 100 μl of 90 % hydrazine hydrate was added. After refluxing for 2 hours, ethanol was removed. The residue obtained was dissolved in 15 ml of 1 % hydrochloric acid, and washed twice with chloroform/isopropanol (5 : 1). After adjustment to pH 8 with conc. ammonia water, the solution was extracted with chloroform/isopropanol (5 : 1) and the extract was subjected to filtration by dehydration with anhydrous sodium sulfate. Then, the filtrate was removed under reduced pressure to give 130 mg of N-(4-aminobutyl)-normorphine (hereinafter abbreviated as ABNM).

An immunogen to mouse was prepared as described below by use of the above ABNM.

After mixing a solution of 15 mg of ABNM dissolved in 0.3 ml of dimethylformamide and a solution of 10 mg of a human immunoglobulin (IgG) which is a macromolecular carrier dissolved in 1.5 ml of pure water, 0.5 ml of an aqueous 10 % solution of 1-cyclohexyl-3-(2-morpholinoethyl)-carbodiimide metho-p-toluenesulfonate (CMEC) was added, the pH was adjusted to pH 5.5, and the mixture was stirred at room temperature for 16 hours. Then, the mixture was dialyzed twice against pure water, and the nondialyzed fractions were lyophilized to give 10 mg of a bound product of NM and IgG (hereinafter abbreviated as NM-IgG) to be used as the immunogen.

The antigen for assay was prepared as described below.

In the same manner as in the above method for preparation of immunogen, except for using bovine serum albumin (BSA) in place of IgG and 1-ethyl-3-(dimethylaminopropyl)carbodiimide hydrochloride (EDPC) in place of CMEC, 11 mg of a bound product of NM and BSA (hereinafter abbreviated as NM-BSA) which is an antigen for assay was obtained.

### Example 2

[Preparation of hybridoma strain producing monoclonal antibody to morphine]

(1) Immunization of mouse and preparation spleen lymphocytes:

A volume 0.4 ml of PBS (Dulbecco's phosphate buffered saline, pH 7.4) containing 400 μg of NM-IgG which is the immunogen prepared in Example 1 dissolved therein and 0.4 ml of Freund's complete adjuvant were thoroughly mixed, and 0.2 ml of the emulsion obtained was administered subcutaneously into the abdomen of BALB/c mouse (female, 8 weeks old).

Two weeks after the initial immunization, 0.2 ml of the emulsion prepared similarly as described above was administered subcutaneously into the abdomen of the above mouse.

Further, two weeks later, 0.5 ml of the emulsion obtained by mixing thoroughly 1 ml of PBS containing 400 μg of the above antigen dissolved therein and 1 ml of Freund's incomplete adjuvant was administered intraperitoneally into the above mouse.

Further, two weeks later, as the final immunization, 0.2 ml PBS of 100 μg of the above antigen dissolved therein was administered into the tail vein of the above mouse.

From the mouse thus immunized, the spleen was removed on the day 3 from the final immunization was washed in a laboratory dish containing MEM (a solution of medium powder for lymphocyte cultivation dissolved in distilled water, containing 10 mM HEPES), transferred into another laboratory dish containing 10 ml of MEM and loosened with the use of the frost portion of a sterilized slide glass.

The suspended lymphocytes thus obtained was suspended in MEM (37 °C), and washed by centrifugation (rotational number: 1,400 rpm, time: 6 minutes). After this operation was repeated further twice, the lymphocytes were resuspended in MEM (37 °C) to give mouse spleen lymphocytes to be provided for cell fusion.


(2) Cell fusion:

Mouse 8-azaguanine resistant myeloma cells (X-63-Ag, 653;653) in the logarithmic growth phase were recovered, and the cells obtained by centrifugation (rotational number: 1,400 rpm, time: 6 minutes) were suspended in MEM (37 °C) and washed by centrifugation (rotational number, 1,400 rpm, time: 6 minutes). This operation was repeated further once, and then the cells were resuspended in MEM (37 °C) to give mouse myeloma cells to be provided for cell fusion.

$4.70 \times 10^7$ of the above mouse myeloma cells and $2.35 \times 10^8$ of the above mouse spleen lymphocytes were placed in a conical centrifugal tube of 50 ml volume made of a plastic, mixed together and centrifuged (rotational number, 1,400 rpm; time: 6 minutes) then the supernatant was removed, followed by loosening of the pellet by lightly tapping the same centrifugal tube.

While rotating the same centrifugal tube, 1 ml of 40 % PEG 1500 solution (37 °C) was added over one minute, and the reaction was carried out for additional one minute while rotating the tube.

Next, while rotating the tube, MEM (37 °C) was gradually added, finally to 10 ml, and centrifuged (rotational number: 800 rpm, time:·6 minutes), then the supernatant was removed.

The pellet was loosened by tapping lightly the same centrifugal tube, resuspended in 140 ml of HAT medium (RPMI1640 medium containing $1 \times 10^{-4}$ hypoxanthine, $4 \times 10^{-7}$ aminopterin, $1.6 \times 10^{-5}$ thymidine, 0.2 U/ml of bovine insulin and 20 % fetal bovine serum, incubated at 37 °C), apportioned each in 100 μl into each culture well of 18 sheets of 96-well culturing plates and cultivated by use of a $CO_2$ incubator (5 % $CO_2$, 95 % air, 37 °C, humidity 100 %).


(3) Selection of hybridoma:

Over 2 to 4 weeks from initiation of the cultivation as described above (2), it was investigated according to the ELISA method shown below whether the antibody to morphine was contained in the culture supernatant of each well of the culturing plate in which cell growth was recognized.

First, into each assay well of a 96 well U-bottomed ELISA plate (hereinafter abbreviated as ELISA plate) was apportioned each 50 μl of a NM-BSA solution (2 μg/ml, dissolved in 0.05 M carbonate buffer of pH 9.8) which is the antigen for assay prepared in Example 1, and left to stand at 4 °C overnight (by such a treatment, NM-BSA is adsorbed nonspecifically onto the contact surface of each assay well).

Next, each assay well was washed with a washing solution (PBS containing 0.05 % Tween 20 (trade name); hereinafter abbreviated as PBS-Tween), and each 100 μl of 0.5 % BSA solution (dissolved in PBS) was apportioned into each assay well, followed by leaving to stand at room temperature for 30 minutes. These respective assay wells were washed with PBS-Tween, and the culture supernatant of each culture well of the above culturing plate was apportioned in each 50 μl in each·assay well and left to stand at room temperature for 2 hours (as a negative control, HAT medium was used; on the other hand, as a positive control, the serum of mouse used for cell fusion in the present invention diluted to 100-fold with PBS-Tween was used).

As the next step, the above each assay well was washed with PBS-Tween, and the alkaline phosphatase-labelled antibody solution to mouse immunoglobulin was apportioned in each 50 μl into each assay well, and left to stand at room temperature for one hour. Each assay well was washed with PBS-Tween, and then each 100 μl of sodium p-nitrophenylphosphate·6H₂O solution (1 mg/ml) was apportioned into each assay well, incubated at room temperature for 30 minutes, followed by measurement of absorbance of each assay well at 405 nm by microplate spectrophotometer for the assay plate.

As the result of such investigations, production of antibody to morphine was recognized in 14 among 1013 culture wells in the culturing plate.

For the 14 culture wells having produced antibody, inhibition tests were conducted by use of morphine (the same operation was conducted as in the above ELISA except for placing at the same time 25 μl of the supernatant of the culture well and 25 μl of PBS-Tween containing 10 μg of morphine dissolved therein in the assay well in place of 50 μl of the supernatant of the culture well).

As the result, 5 culture wells containing antibody inhibited by morphine were recognized among 14. Thus, in the 5 culture wells, hybridomas producing antibody to morphine were confirmed to exist.

(4) Cloning of hybridoma:

By use of RPMI1640 medium containing 20 % FCS, hybridomas in the five culture wells (A, B, C, D and E) confirmed of antibody production shown in the above step (3) were cloned according to the limiting dilution method.

For culture, a 96-well culturing plate was employed, and by use of thymus cell suspension (10⁷/ml) of BALB/c mouse as the feeder cell, cultivation was carried out at (hybridoma 1 to 5)/(thymus cell suspension 100 μl)/well, and each 100 μl of RPMI1640 medium containing 20 % FCS was added on the day 5 after initiation of the cultivation, and further cultivation was continued.

In cloning of hybridomas in the above 5 culture wells of A, B, C, D and E, the supernatant of the culture well observed as single colony on the day 10 to 14 was collected and subjected to screening of antibody producing well according to the ELISA method (the same method as in the above step (3)). Thus, at least one strain or more of hybridomas were obtained respectively from the culture wells of A, B, C, D and E. Among them, in view of cell growth and the amount of antibody produced, each one strain of hybridomas derived from cultured wells of A, B, C, D and E was selected and subjected to recloning.

The strains thus obtained are called MO-2 strain (FERM-P No. 1910), MO-3 strain (FERM-P No. 1911), MO-4 strain, MO-5 strain (FERM-P No. 1912), MO-6 strain, and the monoclonal antibodies produced by these cells are called MO-2, MO-3, MO-4, MO-5 and MO-6, respectively.

The class·subclass and the type of L-chain of the monoclonal antibody of these 5 strains contained in the culturing supernatant were determined in the following assay test I, and reactivities with various compounds investigated in the assay test II.

Assay test I

[Determination of class·subclass of monoclonal antibody to morphine]

Determination of class·subclass of the immunoglobulins produced by MO-2, MO-3, MO-4, MO-5 and MO-6 strains was conducted according to the same ELISA method as in the above step (3) by use of specific peroxidase-labelled antibody solutions for respective class·subclasses of mouse antibody (antibodies labelled with horseradish peroxidase to IgG₁, IgG₂ₐ, IgG₂ᵦ, IgG₃, Igm, IgA, x type L-chain or λ type L-chain), and the ouchterlony method by use of specific antibody solutions specific for respective class·subclasses of mouse antibodies (antibodies to IgG₁, IgG₂ₐ, IgG₂ᵦ, IgG₃, IgM, IgA, x type L-chain or λ type L-chain).

As the result, the monoclonal antibody (MO-2) produced by the MO-2 strain was found to be an antibody belonging to IgG₁ having x type L-chain, the monoclonal antibody (MO-3) produced by the MO-3 strain an antibody belonging to IgG₂ᵦ having x type L-chain, the monoclonal antibody (MO-4) produced by the MO-4 strain an antibody belonging to IgA having x type L-chain, the monoclonal antibody (MO-5) produced by the MO-5 strain an antibody belonging to IgG₁ having λ type L-chain and the monoclonal antibody (MO-6) produced by the MO-6 strain an antibody belonging to IgM having λ type L-chain.

Assay test II

[Investigation of reactivities of monoclonal antibodies to morphine with various compounds]

Concerning reaction specificities of monoclonal antibodies of MO-2, MO-3, MO-4, MO-5 and MO-6, reactivities with metabolites of morphine in living body [morphine-3-glucronide (M-3-G) or morphine-6-glucronide (M-6-G), or other compounds having analgesic effects [cocaine, codeine, dihydrocodeine, ethylmorphine, phentanil or methadone] were investigated according to the same ELISA method as described above in the step (3) (however, in place of 50 μl of the hybridoma supernatant, a mixture of 25 μl of a compound solution diluted in multiple stages with PBS-Tween and 25 μl of a solution of any of the above monoclonal antibodies was used).

The results of reactivity of the respective monoclonal antibodies with the respective compounds are shown in reactivity ratio with morphine in Table 1.

Table 1

| Compound | MO-2 | MO-3 | MO-4 | MO-5 | MO-6 |
|---|---|---|---|---|---|
| Morphine | 100 | 100 | 100 | 100 | 100 |
| Cocaine | < 1.0 | < 0.001 | < 0.001 | < 0.001 | < 0.1 |
| Codeine | < 1.0 | < 0.1 | < 0.1 | < 0.1 | < 0.1 |
| Dihydrocodeine | < 1.0 | < 0.1 | < 0.1 | < 0.1 | < 0.1 |
| Ethylmorphine | < 1.0 | < 0.01 | < 0.01 | < 0.01 | < 0.1 |
| Fentanyl | < 1.0 | < 0.01 | < 0.001 | < 0.001 | < 0.1 |
| Methadone | < 1.0 | < 0.001 | < 0.001 | < 0.001 | < 0.1 |
| M - 3 - G | 2.0 | 0.1 | 0.2 | 0.5 | 0.3 |
| M - 6 - G | 440 | 0.6 | 0.9 | 0.9 | 1.0 |

Example 3

[Production of monoclonal antibody to morphine by flask cultivation]

Cultured cells of MO-3 strain obtained by cultivation in RPMI1640 medium containing 15 % FCS were transferred into 10 ml of RPMI1640 medium (containing no FCS) and cultured immediately before substantially all the cells were dead.

The monoclonal antibody to morphine (MO-3) was contained in an amount of 50 μg/ml (measured by the one dimensional plate immunodiffusion method) in the supernatant obtained by centrifugation of the culture broth (rotational number: 3000 rpm, time: 5 minutes).

Example 4

[Production of monoclonal antibody to morphine intraperitoneally in mouse]

For obtaining a large amount of monoclonal antibody to morphine, cells of the MO-3 strain were cultured intraperitoneally in mouse.

Intraperitoneally into a BALB/c mouse (female, 8 weeks old, administered 2 weeks before intraperitoneally with 0.5 ml of pristane) was administered the whole amount of 0.5 ml of PBS having $2 \times 10^6$

14

cells of the MO-3 strain suspended therein.

The body weight of the mouse exhibited remarkable increase at around one week after, and ascites was collected with a syringe of 19G on the days 7, 9 and 11 after cell administration (total collected amount: 6 ml/mouse). The ascites was centrifuged (rotational number: 3,000 rpm, time: 5 minutes) to obtain ascites supernatant.

The monoclonal antibody to morphine (MO-3) was found to be contained in the ascites supernatant in an amount of 10 mg/ml (measured by the one dimensional plate immunodiffusion method).

Reference example 1

[Production and purification of antibody]

Production of a monoclonal antibody having high specificity for morphine and normorphine produced by the hybridoma strain of the MO-3 strain (FERM-P No. 1911) was carried out as described below.

The strain cells in number of $10^7$ suspended in phosphate buffer were intraperitoneally administered into BALB/c mouse (male, 8 weeks old, administered 2 weeks before intraperitoneally with 0.5 ml of pristane). Remarkable increase of mouse body weight was recognized at around the week 1, and ascites was taken out suitably on the weeks 1 to 3. The antibody value of the monoclonal antibody thus obtained was found to be $10^6$ to $10^8$.

Purification of the monoclonal antibody from the ascites obtained was performed as described below.

The above ascites was dialyzed against Tris-HCl buffer (pH 7.4) and passed through a DEAE-cellulose column equilibrated with the same buffer. The fractions passed as such were salted out with 50 % saturated ammonium sulfate, and the precipitates obtained were dissolved in phosphate buffer (pH 7.4) and dialyzed against the same buffer.

The purity of the monoclonal antibody having a reactivity with high specificity for morphine and normorphine thus obtained was found to be high purity as determined by slab gel electrophoresis by use of SDS polyacrylamide gel.

Example 5

[Preparation of "normorphine carrier"]

To a solution of 271 mg of normorphine dissolved in 20 ml of dioxane were added a solution of 340 mg of N-(4- bromobutyl)phthalimide dissolved in 5 ml of ethanol and 600 mg of sodium carbonate were added, and subsequently the mixture was heated under reflux for 20 hours. After filtration of inorganics from the reaction mixture, the filtrate was concentrated to dryness under reduced pressure. The residue obtained was dissolved in an extremely small amount of ethyl acetate and applied onto a silica gel column [20 mm$\phi$x 300 mmL; Kieselgel 60 (70 -200 mesh ASTM)]. This was eluted with 60 ml of ethyl acetate/methanol (9 : 1), and subsequently eluted with ethyl acetate/methanol (5 : 1) and fractionated into each 20 ml. By analysis of TLC (Kieselgel 60; developing solvent: ethyl acetate/methanol/conc. ammonia water = 85 : 10 : 5), the fractions containing only the desired product were collected, followed by evaporation of the solvent under reduced pressure. The residue obtained was dissolved in 20 ml of ethanol, and then refluxed with addition of 100 μl of 90 % hydrazine hydrate for 2 hours, followed by evaporation of ethanol. The residue obtained was dissolved in 15 ml of 1 % hydrochloric acid, and then washed twice with chloroform/isopropanol (5 : 1). After adjustment to pH 8 with conc. ammonia water, the mixture was extracted with chloroform/isopropanol (5 : 1), dehydrated over anhydrous sodium sulfate and filtered. Then, the filtrate was evaporated under reduced pressure to give 130 mg of N-(4-aminobutyl)normorphine (hereinafter abbreviated as ABNM).

The bound product of normorphine with BSA which is a macromolecular protein (abbreviated as normorphine-BSA) was prepared by use of the above ABNM as described below.

After a solution of 15 mg of ABNM dissolved in 0.3 ml of dimethylformamide and a solution of 10 mg of BSA which is a macromolecular carrier dissolved in 1.5 ml of pure water were mixed, 0.5 ml of an aqueous 10 % solution of 1-ethyl-3-(dimethylaminopropyl)carbodiimide-hydrochloride (EDPC) was added, and after adjustment to pH 5.5, the mixture was stirred at room temperature for 16 hours, then dialyzed twice against pure water, and the nondialyzed fractions were lyophilized to give 10 mg of normorphine-BSA to be used as the immunogen.

The method for carrying normorphine on a carrier through BSA of normorphine-BSA in preparation of the "normorphine carrier" was practiced as described below.

A mixture of 100 μl of a normorphine-BSA solution (400 μg/ml) and 100 μl of a polystyrene latex (solid component 4.0 % by weight, particle size 0.8 μm in diameter, solvent 0.1 M phosphate buffer of pH 7.4) was shaken at 37 °C for 3 hours, to have normorphine-BSA physically carried on the polystyrene latex surface to give a "normorphine carrier" suspension.

Example 6

[Preparation of "normorphine carrier"]

Half of the "normorphine carrier" suspension of Example 5 was further heated at 37 °C for 5 hours to obtain a "normorphine carrier" suspension.

Example 7

[Preparation of "antibody carrier"]

The "antibody carrier" was obtained by mixing 100 μl of the monoclonal antibody solution (1 mg/ml, solvent: 0.1 M phosphate buffer of pH 7.4) exhibiting very high specificities for morphine and normorphine of Reference example 1 and 100 μl of a polystyrene latex (solid component 4.0 % by weight, particle size 0.8 μm in diameter, solvent: 0.1 M phosphate buffer of pH 7.4) which is the carrier, and shaking the mixture at 37 °C for 3 hours, thereby having the monoclonal antibody physically carried on the polystyrene latex surface.

Example 8

[Assay of morphine diluted with urine of healthy man]

The "normorphine carrier" prepared in Example 5 and the "antibody carrier" prepared in Example 7 were added dropwise each in 25 μl on the two adjacent points on the reaction plate at an interval so that these reagents did not contact each other, and each 5 μl of "standard morphine solutions" with morphine concentrations of 0, 0.1, 0.2 or 0.3 ppm prepared by dilution with urine of healthy men (Manufactured by HYLAND DIAGNOSTIC CO.) was added dropwise separated from each other at intervals so as not contact either of the two points of the reagents, followed immediately by stirring and mixing of the "normorphine carrier", the "antibody carrier" and the sample to be measured on the same reaction plate by a stirring rod. When the same plate was continued to be moved slowly, the polystyrene latex particles were observed with naked eyes to be agglutinated at 16 seconds when the morphine concentration of the "standard morphine solution" was 0 ppm, at 22 seconds when it was 0.1 ppm, at 35 seconds when it was 0.2 ppm and at 180 seconds or longer when it was 0.3 ppm.

Example 9

[Measurement of morphine diluted with urine of healthy man]

The "normorphine carrier" prepared in Example 6 and the "antibody carrier" prepared in Example 7 were added dropwise each in 25 μl on the two adjacent points on the reaction plate at an interval so that these reagents did not contact each other, and each 5 μl of "standard morphine solutions" with morphine concentrations of 0, 0.1, 0.2 or 0.3 ppm prepared by dilution with urine of healthy men (Manufactured by

16

HYLAND DIAGNOSTIC CO.) was added dropwise separated from each other at intervals so as not to contact either of the two points of the reagents, followed immediately by stirring and mixing of the "normorphine carrier", the "antibody carrier" and the sample to be measured on the same reaction plate by a stirring rod. When the same plate was continued to be moved slowly, the polystyrene latex particles were observed with naked eyes to be agglutinated at 13 seconds when the morphine concentration of the "standard morphine solution" was 0 ppm, at 19 seconds when it was 0.1 ppm, at 30 seconds when it was 0.2 ppm and at 150 seconds or longer when it was 0.3 ppm.

Comparative example 1

[Assay of cocaine]

Assay was conducted in entirely the same manner as in Example 8 except that the standard solutions prepared with cocaine were used in place of morphine in Example 8.

As the result, polystyrene latex particles were observed with naked eyes to be agglutinated at 16 seconds when the concentration of cocaine was 0 ppm, also at 16 seconds when it was 0.3 ppm.

Comparative example 2

[Assay of ethylmorphine]

Assay was conducted in entirely the same manner as in Example 8 except that the standard solutions prepared with ethylmorphine were used in place of morphine in Example 8.

As the result, polystyrene latex particles were observed with naked eyes to be agglutinated at 16 seconds when the concentration of ethylmorphine was 0 ppm, also at 16 seconds when it was 0.3 ppm.

Comparative example 3

[Assay of cocaine]

Assay was conducted in entirely the same manner as in Example 9 except that the standard solutions prepared with cocaine were used in place of morphine in Example 9.

As the result, polystyrene latex particles were observed with naked eyes to be agglutinated at 13 seconds when the concentration of cocaine was 0 ppm, also at 13 seconds when it was 0.3 ppm.

Comparative example 4

[Assay of ethylmorphine]

Assay was conducted in entirely the same manner as in Example 9 except that the standard solutions prepared with ethylmorphine were used in place of morphine in Example 9.

As the result, polystyrene latex particles were observed with naked eyes to be agglutinated at 13 seconds when the concentration of ethylmorphine was 0 ppm, also at 13 seconds when it was 0.3 ppm.

Example 10

[Preparation of "NM bound protein"]

17

Similarly as in Example 1, 130 mg of ABNM was obtained.

The bound product of normorphine and BSA which is a macromolecular protein (hereinafter abbreviated to as "normorphine-BSA") was prepared by the use of the above ABNM as described below.

After 0.3 ml of dimethylformamide was mixed with 15 mg of ABNM and an aqueous solution of 10 mg of bovine serum albumin (BSA) dissolved in 1.5 ml of water , 0.5 ml of 10 % EDPC was added to adjust pH to 5.5 and after stirred at room temperature for 20 hours, the mixture was dialyzed twice against pure water, followed by lyophilization of the non-dialyzed fraction, to give 11 mg of a normorphine-BSA which was used as an immunogen.

Example 11

[Production and purification of antibody]

Production of a monoclonal antibody having high specificity for morphine and normorphine produced by a hybridoma strain of MO-3 strain (FERM-P No. 1911) was performed according to the method as described in Japanese Patent Application No. 234826/1988.

Production of the monoclonal antibody was performed as described below.

$10^7$ strain cells suspended in PBS were administered intraperitoneally into BALB/c mouse (male, 8 weeks old, administered intraperitoneally with 0.5 ml of pristan 2 weeks before). Marked increase of mouse body weight was recognized after about one week, and ascites were suitably taken out after one to 3 weeks. The monoclonal antibody thus obtained has an antibody titer of $10^6$ to $10^8$.

Purification of the monoclonal antibody from the ascites obtained was conducted as follows.

The above ascites were dialyzed against Tris-buffer (pH 7.4), and flowed through a DEAE-cellulose column equilibrated with the same buffer. The fraction passed was salted out with 50 % saturated ammonium sulfate, and the resultant precipitate was dissolved in PBS and dialyzed against the same buffer. The purity of the monoclonal antibody to MO thus obtained was found to be higher than any antibody according to SDS-polyacrylamide gel electrophoresis.

According to the method for assay MO by use of the purified monoclonal antibody to MO obtained as described above (ELISA method), it has become possible to perform high sensitivity and rapid assay of MO.

Example 12

Preparation of "NM bound protein" and "enzyme-labeled antibody" for MO assay kit

The "NM bound protein" and "enzyme-labeled antibody" for MO assay kit were prepared as described below.

The "NM bound protein" obtained as shown in Example 10 was dialyzed under 4 °C overnight against PBS, apportioned each in 0.5 ml into bottles and stored after lyophilization (100 to 3000 μg/bottle) to provide the "NM bound protein" which is the assay kit of the present invention. During assay of MO, 0.5 ml of distilled water was added into this bottle to dissolve well the lyophilized powder, and diluted with PBS to a desired concentration before use.

The antibody in the "enzyme-labeled antibody" was obtained as the purified monoclonal antibody as shown in Reference example 2 by use of the MO-3 strain (FERM-P 1911).

By use of the monoclonal antibody, the antibody was labeled with an enzyme according to the known method as shown below to prepare the "enzyme-labeled antibody" for the assay kit of the present invention.

First, 7.32 g of horseradish peroxidase was dissolved in 1 ml of distilled water and 200 μl of 0.1 M sodium periodate was added, followed by standing at room temperature for 30 minutes. After the enzyme solution was dialyzed at 4 °C overnight against 1 mM acetate buffer (pH 4.5), 100 μl of 0.2 M sodium carbonate buffer (pH 9.5) was added to adjust pH to 9.5. On the other hand, 8.78 mg of the monoclonal antibody (MO-3) dissolved in PBS was dialyzed at 4 °C overnight against 0.01 M sodium carbonate buffer (pH 9.5). The peroxidase and the monoclonal antibody thus obtained was mixed, left to stand at room temperature for 2 hours and a half, and 200 μl of 0.4 % sodium borohydride solution was added to the reaction mixture, followed by standing at 4 °C for 2 hours. The peroxidase-labeled monoclonal antibody thus obtained was dialyzed at 4 °C overnight against PBS, and this was diluted to 100-fold with 0.1 M PBS (pH 7.4), apportioned each in 0.2 ml into a bottle and stored under lyophilization to provide the "enzyme-labeled antibody" (10 µg/bottle) which is assay kit of MO. During assay of MO, 0.2 ml of distilled water was added into the bottle to dissolve well the lyophilized powder, and the solution was suitable diluted with 0.1 M PBS (pH 7.4) before use.

Example 13

Preparation of calibration curve of MO by use of "enzyme- labeled antibody"

The NM-BSA which is the "NM bound protein" (bound product of normorphine and BSA, 2 µg/ml) was apportioned each in 100 µl into a 96-well flat-bottomed plate for immunoassay which is the solid phase support (microplate made of polystyrene produced by Nunc Co.), and left to stand under 4 °C overnight to immobilize NM-BSA onto each well. Next, for preventing non-specific adsorption of the "enzyme-labeled antibody" onto the plate, a washing solution containing 10 % calf serum (phosphate buffer of pH 7.4 containing 0.05 % of Tween 20) was placed and left to stand under room temperature for 30 minutes. Next, after washing with the same washing solution, 50 µl of the standard MO solution prepared by using the diluted pooled serum of healthy man (Nescol X, trade name, produced by The Chemo-and Sero-Therapeutic Research Institute) to 10-fold with the same washing solution [(0 to 30 ng)/ml] and 50 µl of the "enzyme-labeled antibody" prepared in Example 10 were added at the same time into each well, followed by mixing and standing under room temperature for 30 minutes. Next, after washed with the same washing solution, the "substrate solution" (10 mg of o-phenylenediamine and 10 µl of 35 % $H_2O_2$ dissolved in 25 ml of 50 mM citrate buffer, pH 5.0) was apportioned each in 100 µl and left to stand under light shielding at room temperature for 5 minutes. Finally, each 50 µl of 2N sulfuric acid was apportioned to stop the enzymatic reaction, and absorbance of the solution after stopping of the reaction at 492 nm was assayed by use of a microplate photometer. As the result, the assay method of MO by use of the "enzyme-labeled antibody" could measure MO at high sensitivity in about 40 minutes by use of the 96-well flat-bottomed plate for immunoassay having immobilized the "NM bound protein" (microplate made of polystyrene of Nunc Co.). The results are shown in Fig. 1.

Example 14

Recovery test of MO

MO was assayed in the same manner as in Example 13 except for adding MO hydrochloride to "Nescol X" in Example 13 with an amount of 3 ppb, 10 ppb and 30 ppb in terms of MO, respectively. As the result, the MO amount added and the MO amount assayed were found to be substantially identical. The results are shown in Table 2.

Table 2

| MO concentration in serum after MO added (ng/ml) | Measured results (ng/ml) | | Recovery (%) |
|---|---|---|---|
| | Observed value (reading) | Found value | |
| 0 | 0.051 | 0.51 | - |
| 3 | 0.33 | 3.3 | 96 |
| 10 | 1.1 | 11 | 105 |
| 30 | 3.1 | 31 | 102 |

Example 15

Comparison of measured value of morphine in blood between ELISA method and HPLC

The results of drug monitoring of actual patients (91 men) under morphine analgesic treatment measured by the enzyme-linked immunosorbent assay (ELISA assay) and the results measured by the conventional high performance liquid chromatography (HPLC method) were compared with each other. As the result, it was found to be substantially identical. The results are shown in Fig. 2.

The "monoclonal antibody having a very high specific reactivity with morphine which is an analgesic obtained by culturing of the hybridoma strain" of the present invention can be expected to be utilized for inspection and assay of morphine.

According to the present invention, by use of an assaying kit of morphine comprising essentially the "normorphine carrier" and the "antibody carrier", morphine can be assayed with high specificity, rapidly, at high sensitivity and easily.

**Claims**

1. A monoclonal antibody to morphine produced by the hybridoma strain obtained by cell fusion of mouse lymphocytes and mouse myeloma cells, characterized in that it does not substantially react with codeine but has a very high specific reactivity with morphine.

2. A method for preparing a monoclonal antibody to morphine, which comprises culturing the hybridoma strain obtained by cell fusion of mouse lymphocytes and mouse myeloma cells, characterized in that it does not substantially react with codeine but has a very high specific reactivity with morphine.

3. The hybridoma strain obtained by cell fusion of mouse lymphocytes and mouse myeloma cells, characterized in that it does not substantially react with codeine but has a very high specific reactivity with morphine.

4. An assaying kit of morphine comprising essentially:

(a) a carrier having an antibody having a very high specific immunoreactivities with morphine and normorphine carried thereon; and

(b) a carrier having normorphine carried thereon.

5. An assaying method of normorphine, which comprises reacting morphine in a sample to be assayed and normorphine carried on a carrier competitively with the antibody carried on the carrier by use of the assaying kit of morphine composed essentially:

(a) a carrier having an antibody having a very high specific immunoreactivities with morphine and normorphine carried thereon; and

(b) a carrier having normorphine carried thereon.

FIG. 1

EP 0 363 041 A1

# FIG. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,D | MOLECULAR IMMUNOLOGY, vol. 20, no. 12, 1983, pages 1419-1422, Pergamon Press Ltd, Oxford, GB; J.A. GLASEL et al.: Properties of murine anti-morphine antibodies"<br>* P. 1421, Table 1, immunoglobulin 12D4 * | 1-3,5 | G 01 N  33/94<br>G 01 N  33/577<br>C 12 P  21/00 |
| Y,D | EUROPEAN JOURNAL OF PHARMACOLOGY, vol. 38, 1976, pages 149-156, North-Holland Publishing Company, Amsterdam, NL; A.R. GINTZLER et al.: "Radioimmunoassay for the simultaneous determination of morphine and codeine"<br>* P. 153, column 1, 1 22 - column 2, 1. 3; Table 1 * | 1-5 | |
| Y,D | MOLECULAR IMMUNOLOGY, vol. 20, no. 9, 1988, pages 937-943, Pergamon Press plc, Oxford, GB; J.I. SAWADA et al.: "Production and characterization of high-affinity monoclonal antibodies against morphine"<br>* p. 938, column 1, 1. 7 - p. 939, column 1, 1. 21 * | 1-5 | **TECHNICAL FIELDS SEARCHED (Int. Cl.5)**<br><br>G 01 N<br>C 12 P |
| Y,D | RESEARCH COMMUNICATIONS IN CHEMICAL PATHOLOGY AND PHARMACOLOGY, vol. 17, no. 4, August 1977, pages 595-603, PJD Publications Ltd, Westbury, N.Y., US; J.W.A. FINDLAY et al.: "Specific radioimmunoassays for codeine and morphine. Metabolism of codeine to morphine in the rat"<br>* p. 600, Table 1 *<br>---             -/- | 1-3,5 | |

The present search report has been drawn up for all claims

| Place of search | Date of completion of the search | Examiner |
|---|---|---|
| THE HAGUE | 27-12-1989 | VAN BOHEMEN C.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

## EUROPEAN SEARCH REPORT

**European Patent Office**

### DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | LIFE SCIENCES, vol. 36, no. 26, 1985, pages 2523-2529, Pergamon Press Ltd., N.Y., US; J.A. GLASEL et al.: "Rabbit anti-idiotypic antibodies raised against monoclonal anti-morphine IgG block opiate receptor sites" * p. 2523, l. 26-30 * | 1-3,5 | |

TECHNICAL FIELDS SEARCHED (Int. Cl.5)

The present search report has been drawn up for all claims

| Place of search | Date of completion of the search | Examiner |
|---|---|---|
| THE HAGUE | 27-12-1989 | VAN BOHEMEN C.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)